# EUROPEAN PATENT APPLICATION

(11) **EP 4 209 219 A1**
(43) Date of publication of application: **12.07.2023**
(21) Application number: 22000007.9
(22) Date of filing: 07.01.2022
(51) Int. Cl.: A61K 31/58, A61K 31/502, A61P 11/06, A61P 5/44, A61P 29/00, A61P 37/00

(54) **COMBINATION OF BUDESONIDE AND 5-AMINO-2,3-DIHYDRO-1,4-PHTALAZINEDIONE**

(71) Applicant: MetrioPharm AG, 8002 Zürich (CH)
(72) Inventor: The designation of the inventor has not yet been filed

(57) **Abstract**

The present invention relates to a combination of budesonide and 5-amino-2,3-dihydro-1,4-phthalazinedione or one of its pharmaceutically acceptable salts. This pharmaceutical combination shall be used in the treatment of chronic inflammatory diseases, especially COPD, asthma, Crohn's disease, colitis ulcerosa and autoimmune hepatitis. The invention relates in particular to the use of 5-amino-2,3-dihydro-1,4-phthalazinedione sodium salt in this combination. Pharmaceutical compositions, advantageous formulation techniques and a method of treatment are disclosed.

## Description

### FIELD OF THE INVENTION

The present invention relates to a combination of budesonide and 5-amino-2,3-dihydro-1,4-phthalazinedione or one of its pharmaceutically acceptable salts. This pharmaceutical combination shall be used in the treatment of chronic inflammatory diseases, especially COPD, asthma, Crohn's disease, colitis ulcerosa and autoimmune hepatitis. The invention relates in particular to the use of 5-amino-2,3-dihydro-1,4-phthalazinedione sodium salt in this combination. Pharmaceutical compositions, advantageous formulation techniques and a method of treatment are disclosed.

### BACKGROUND OF THE INVENTION

Glucocorticoids are widely used in the treatment of inflammatory diseases, for example in the treatment of rheumatoid arthritis, osteoarthritis, gouty arthritis, ulcerative colitis, multiple sclerosis, asthma, severe psoriasis, some tumors, cerebral edema, chemotherapy induced nausea and vomiting, to prevent transplant rejection, and any further indication that requires a fast and considerable anti-inflammatory and/or immunosuppressive effect.

Over the last four decades budesonide has become one of the most prescribed glucocorticoids in several medical fields. Available dosage forms include an inhaler, a nebulization solution, pills, a nasal spray and rectal forms. Inhalatory dosage forms are used in the long-term management of asthma and chronic obstructive pulmonary disease (COPD). The pills in a delayed release form and rectal forms are used in the treatment of inflammatory bowel diseases including Crohn's disease, ulcerative colitis, and microscopic colitis. The nasal spray is used for allergic rhinitis and nasal polyps. Budesonide showed good results in the inhalatory treatment of COVID-19 patients.

In general, budesonide has a positive impact on the course of many diseases and conditions related to the immune system. On the other hand, long-term administration of budesonide comes along with numerous serious side effects, many of them typical for glucocorticoids. Adverse drug reactions with the inhaled form include amongst others respiratory infections, cough and headaches. Common side effects with oral dosage forms include an increased risk of infection, loss of bone strength and cataracts. When used over long time budesonide may cause adrenal insufficiency (cf. www.drugs.com/monograph/budesonide-systemic-oral-inhalation.html, as of September 17^{th}, 2021).

Therefore, there is a medical need to provide a pharmaceutical drug that helps to spare budesonide, i.e. to reduce the required dosage of budesonide without reducing its desired effectiveness.

It is also a task of the present invention to provide pharmaceutical drug that allows to avoid and/or reduce adverse side effects of budesonide.

Surprisingly, this task is solved by the pharmaceutical combination of budesonide and 5-amino-2,3-dihydro-1,4-phthalazinedione or one of its pharmaceutically acceptable salts.

The pharmaceutical combination of budesonide and 5-amino-2,3-dihydro-1,4-phthalazinedione sodium salt showed to be particularly effective.

### DESCRIPTION OF THE INVENTION

Budesonide is a synthetic glucocorticoid with a weak mineralocorticoid activity. It is pharmaceutically used for the local treatment of asthma bronchiale, COPD (chronic obstructive pulmonary disease), nasal polyps, inflammatory bowel diseases such as Crohn's disease and colitis ulcerosa, eosinophilic esophagitis and chronic liver diseases such as autoimmune hepatitis. Recently, it could also be shown that inhalatory application of budesonide could reduce the number severe COVID-19 cases, in comparison to control (Yu et al. (2021) Lancet 398: 843-855).

Budesonide is a diastereomeric mixture of (22R)- and (22S)-11β,21-dihydroxy-16α,17α-(butylidenebis(oxy)pregna-1,4-diene-3,20-dione. The IUPAC name is (1S,2S,4R,8S,9S,11S,12S,13R)-11-hydroxy-8-(2-hydroxyacetyl)-9,13-dimethyl-6-propyl-5,7-dioxapentacyclo[10.8.0.0^{2,9}.0^{4,8}.0^{13,18}]icosa-14,17-dien-16-one. The (22R) isomer is also known as dexbudesonide. In the scope of the present disclosure the term budesonide shall cover the diastereomeric mixture as well as the single isomers.

Dosage forms hitherto commercialized for budesonide are nose drops, nose spray, inhalatory nebulization solutions for the bronchial system, capsules, pills, creams, intravenous injection solutions and enema. Systemic application, however, is limited by a strong first-pass effect.

As all glucocorticoids, budesonide may have immunosuppressive effects with an increased risk of infections. Common side effects encompass headache, dizziness, runny nose, sneezing, coughing, nausea, indigestion, abdominal pain, gas, vomiting, fatigue, back pain, pain, itching, skin rash, fever, swelling of face and neck, difficulty in breathing, severe headache, acne, bruising (cf. MedlinePlus, as of Sep. 29^{th}, 2021). With inhaled budesonide fungal infections of the oral cavity (e.g., candidiasis) and hoarseness may occur.

Numerous drug-drug-interactions need to be considered when using budesonide. The most common interactions that have been reported are with low strength acetylsalicylic acid, metoprolol, fluticasone/salmeterol, arformoterol, budesonide/formoterol, albuterol and albuterol/ipratropium (cf. www.drugs.com/drug-interactions/budesonide.html, as of September 17^{th}, 2021). Interactions with voriconazole are presumed.

5-amino-2,3-dihydro-1,4-phthalazinedione (luminol) belongs to the pharmaceutical class of the phthalazinediones. Compounds of this class are known for their beneficial anti-inflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2017/202496; WO 2018/082814: a.o.).

While most conventional immunomodulatory drugs show serious adverse reactions, or are at least problematic in long-term treatment, 5-amino-2,3-dihydro-1,4-phthalazinedione and its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

In particular, the present application discloses 5-amino-2,3-dihydro-1,4-phthalazinedione or one of its pharmaceutically acceptable salts for use in the treatment of a congenital muscular dystrophy, wherein the pharmaceutically acceptable salt of 5-amino-2,3-dihydro-1,4-phthalazinedione is 5-amino-2,3-dihydro-1,4-phthalazinedione sodium salt.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes. In the scope of the present disclosure all hydrates and solvates shall be included in the term "5-amino-2,3-dihydro-1,4-phthalazinedione or one of its pharmaceutically acceptable salts".

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

The use of anhydrous Form I of 5-amino-2,3-dihydro-1,4-phthalazinedione sodium salt is preferred.

5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

Thus, the present patent application refers also to the use according to the disclosure of all crystalline forms and polymorphs thereof of 5-amino-2,3-dihydro-1,4-phthalazinedione and its pharmaceutically acceptable salts. The use of Form II of 5-amino-2,3-dihydro-1,4-phthalazinedione is preferred.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the disclosure.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts.

As used throughout the present application the term "5-amino-2,3-dihydro-1,4-phthalazinedione or one of its pharmaceutically acceptable salts" shall encompass all the aforementioned molecular variants of 5-amino-2,3-dihydro-1,4-phthalazinedione i.e., 5-amino-2,3-dihydro-1,4-phthalazinedione or one of its pharmaceutically acceptable salts or solvates, hydrates, crystalline polymorphs or tautomers thereof.

A combination of budesonide and 5-amino-2,3-dihydro-1,4-phthalazinedione sodium salt is tested in a cell culture model of Raw264.7 cells, an established *in vitro* model for the testing of the effects of pharmaceutical agents in inflammation (Frazier-Jessen et al. (2002) FEBS Lett 513: 203-207). Because of the involvement of immunocompetent cells it is assumed that the results in this model are predictive not only *in vitro* but for all chronic inflammatory diseases.

The administration of a combination of budesonide and 5-amino-2,3-dihydro-1,4-phthalazinedione sodium salt leads to a significant reduction in IL-6 release (see Example 1). IL-6 (interleukin 6) is a cytokine secreted by macrophages in response to specific microbial molecules (lipopolysaccharides (LPS) in Example 1, a characteristic molecule of the outer membrane of gram-negative bacteria).

To avoid ambiguities in the art, the following terms of interaction among drugs are defined. They are used in this sense throughout the disclosure. If at least two substances (e.g., pharmaceutical agents) are administered and at least one of these substances affects the activity of the other at least one substance a so-called drug-drug interaction is given. If said interaction leads to an exaggerated or increased effect of the drug substance the effect is synergistic. The respective equation can be displayed as (A+B) > A or (A+B) > B, wherein A and B are the percentual or fractional effects (i.e., a value between 0 and 1) seen when the respective substances are administered alone and (A+B) is the percentual or fractional effect seen after combinatory application, respectively. A final effect equal to the sum of the effects seen in the single drug applications is an additive synergistic effect. If the final effect is greater than said expected effect the term supraadditivity is used. Smaller effects are subadditive. If, on the other hand the drug-drug interaction leads to a decreased effect of the drug substance the effect is not synergistic at all, but antagonistic.

Hence, in the scope of this disclosure the terms "antagonism" and "antagonistic" are used for any decreased drug effect induced by drug-drug-interaction, whilst the terms "synergy" and "synergistic" are used for any increased drug effect induced by drug-drug-interaction. To describe and determine the grade of said synergy the terms "subadditive", "additive" and "supraadditive" as well as the respective nouns are used. Hence, the term supraadditivity is used to describe an effect of two or more combined agents that is greater than the expected additive effects of the single agents.

The use of synergistic acting drug combinations can both help to increase the therapeutic efficacy and the potency, wherein the latter primarily helps to reduce off-target toxicity.

The identification of drug-drug-interactions depends on the "no-interaction" null-hypotheses, which is based on the observed drug response and not on any model of mechanism. Hence, for two different drugs, their grade of additivity is based on a point of reference for the readout wherein the point of reference depends on the mathematical model chosen. Various methods exist to identify such interactions and to calculate the expected additive effect of two substances and thus to determine if the actual synergistic effect observed is subadditive, additive or supraadditive. Different methods are recommended in the art. The most common methods are outlined below.

Basic methods like building simple arithmetic sums or the fractional product method provide an easy way to gain first insights if a specific combination has supraadditive potential.

The simple arithmetic sum method for additivity is based on the equation A + B = (A+B), wherein A and B again are the percentual or fractional effects seen when the respective substances are administered alone and (A+B) is the percentual or fractional effect seen after combinatory application of said substances using the same doses as in the single applications. If A + B > (A+B) supraadditivity is given. An obviously striking disadvantage of this method is that results wherein A + B ≥ 100% cannot be analyzed for supraadditivity. Hence, this method is primarily used to interpret combinations of suboptimal doses.

The fractional product method is based on the equation 1 - (1-A)*(1-B) = (A+B). Here, A and B are the fractual effects seen when the respective substances are administered alone and (A+B) is the fractual effect seen after combinatory application of said substances using the same doses as in the single applications. If 1 - (1-A)*(1-B) > (A+B), supraadditivity is given. More sophisticated methods include the use of a so-called isobologram, a graph constructed on a coordinate system defined by individual drug doses showing a "line of additivity" that allows to distinguish between subadditive, additive (along the line) and supraadditive effects. The "line of additivity" connects those single drug doses which display the same effect (e.g., 50% inhibition of a specific marker). All possible dose combinations along this line are expected to show the same effectiveness. Dose combinations located within the triangle built by coordinates and the line of additivity i.e., nearer to the arbitrary point, displaying the same effect are considered as supraadditive. Dose combinations located outside the triangle are considered as subadditive. Respective figures can be mapped using specific software as e.g., CompuSyn (Chou TC and Martin N. ComboSyn, Inc. Paramus, NJ 2007 [www.combosyn.com]).

Also, further specific indicator values as for example the Combination Index (CI, equation of Chou and Talalay (1984) Adv Enzyme Reg 22: 27-55) and the Dose-Reduction Index (DRI, Chou equation 1984) can be calculated easily using the beforementioned specific software. Both these values allow to determine if drug substances act supraadditive synergistically when administered contemporarily. The CI is based on the principles of mass action law and is applicable for any kind of drug combination regardless of mechanism of action, dynamic order or the quantity units used for each drug in the combination. The CI value defines synergism as supraadditive if CI<1 and as additive if CI =1. If CI > 1 the effect is either subadditive or antagonistic. Hence, a CI of 1 also refers to the "line of additivity" In the classic isobologram as mentioned above. In a simplified approach it could be calculated as follows: CI = A(t)/A(x) + B(t)/B(x), wherein A(t) respectively B(t) is the dose of drug A respectively B alone that inhibits x %, whereas A(x) and B(x) stand for the portion of the respective drug in the combination that also inhibits x %. The DRI is a measure of how many folds the dose of each drug in a synergistic combination may be reduced at a given effect level compared with the doses of each drug alone. Therefore, DRI=1 indicates additivity, whereas DRI>1 and <1 indicate supraadditivity and subadditivity (or antagonism), respectively. For displaying purposes, the isobologram, i.e., the equi-effective curve at various concentrations or doses of two drugs as mentioned above, is a dose-oriented figure approach based on a special case of the CI equation. A more convenient figure approach, however, is the effect oriented so-called FaCI plot, displaying the Combination Index (CI) against the fractual effect (Fa), preferably for a specific dose combination.

It is thus found that the administration of a 5-amino-2,3-dihydro-1,4-phthalazinedione or one of its pharmaceutically acceptable salts in addition to a budesonide therapy can reduce both the dosage budesonide without reducing its effectiveness, as well as decrease unwanted effects associated to budesonide.

Particularly, the administration of 5-amino-2,3-dihydro-1,4-phthalazinedione sodium salt in addition to budesonide allows to reduce the dosage of budesonide, as it is found that 5-amino-2,3-dihydro-1,4-phthalazinedione sodium salt and budesonide act in a supraadditive manner when administered concomitantly (see Example 1).

The present application refers thus to a pharmaceutical combination of budesonide and 5-amino-2,3-dihydro-1,4-phthalazinedione or one of its pharmaceutically acceptable salts for use for glucocorticoid-sparing.

Particularly, the administration of 5-amino-2,3-dihydro-1,4-phthalazinedione sodium salt in addition to budesonide allows to decrease unwanted side effects associated to budesonide, since it can be shown that the concomitant administration of 5-amino-2,3-dihydro-1,4-phthalazinedione sodium salt and budesonide reduces unwanted effects related to budesonide.

The addition of 5-amino-2,3-dihydro-1,4-phthalazinedione or one of its pharmaceutically acceptable salts to an already ongoing budesonide therapy can also help to avoid unwanted budesonide effects and can further reduce those unwanted budesonide effects that already have emerged.

In particular, the administration of 5-amino-2,3-dihydro-1,4-phthalazinedione sodium salt in addition to a budesonide therapy can avoid and/or reduce unwanted budesonide effects.

The present application refers thus to a pharmaceutical combination of budesonide and 5-amino-2,3-dihydro-1,4-phthalazinedione or one of its pharmaceutically acceptable salts for use for reducing or avoiding unwanted budesonide side effects.

These unwanted effects of budesonide are similar to those of glucocorticoids in general. They comprise, without being limiting, hypertonia, weight gain and obesity (particularly truncal obesity, including the so called "buffalo hump"), edema, puffiness of the face (moon face), potassium loss, muscle weakness, headache, facial hair growth (in women), thinning of the skin, easy bruising and slow wound healing, glaucoma, cataracts, stomach and duodenum ulcers, irregular menstrual cycle, steroid induced diabetes, loss of control in existing diabetes, osteoporosis (resulting in bone fractures), adrenal joint necrosis (in particular of the hip or the knee joint), psychiatric disturbances (e.g. depression, euphoria, insomnia, mood swings, personality changes), psychotic behavior, growth retardation in children, convulsions, an increased rate of infections, exacerbation of opportunistic infections (such as tuberculosis, *Herpes zoster* and *Pneumocystis* pneumonia), reduced effectiveness of antibiotics and vaccines, and in particular Cushing's syndrome, wherein the latter represents a collection of signs and symptoms that occur due to prolonged exposure to glucocorticoids including for example high blood pressure, abdominal obesity, striae, "moon face", corticosteroid-induced lipodystrophy (e.g. buffalo hump), weak muscles, weak bones, fragile skin, acne, facial hair growth (in women) and irregular menstruation.

A reduction of the dosage of budesonide by adding 5-amino-2,3-dihydro-1,4-phthalazinedione or one of its pharmaceutically acceptable salts to a budesonide therapy also helps to avoid and/or reduce unwanted effects associated to budesonide removal. Typical unwanted effects associated to glucocorticoid removal such as adrenal insufficiency or even an acute Addisonian crisis including symptoms like nausea, vomiting and shock can thus be avoided or at least mitigated.

In particular, the present disclosure relates to a pharmaceutical combination of budesonide and 5-amino-2,3-dihydro-1,4-phthalazinedione or one of its pharmaceutically acceptable salts for use in medicine. 5-amino-2,3-dihydro-1,4-phthalazinedione sodium salt is preferred.

The term chronic inflammatory diseases refers to all disorders characterized by a prolonged inflammation. They entail a progressive shift in the type of cells present at the site of inflammation, such as mononuclear cells, and is characterized by simultaneous destruction and healing of the tissue from the inflammatory process. Reasons for a chronic inflammation can be a failure of eliminating an agent that causes acute inflammation such as infectious organisms including *Mycobacterium tuberculosis,* protozoa, fungi, and other parasites that can resist host defenses and remain in the tissue for an extended period, or the exposure to a low level of a particular irritant or foreign material that cannot be eliminated by enzymatic breakdown or phagocytosis in the body including substances or industrial chemicals that can be inhaled over a long period, for example, silica dust, or they are due to an autoimmune disorder in which the immune system recognizes the normal component of the body as a foreign antigen, and attacks healthy tissue giving rise to diseases such as rheumatoid arthritis (RA) and systemic lupus erythematosus (SLE), or there is a defect in the cells responsible for mediating inflammation leading to persistent or recurrent inflammation, such as auto-inflammatory disorders (Familial Mediterranean Fever), or there are recurrent episodes of acute inflammation. A common denominator of chronic inflammation is oxidative stress and mitochondrial dysfunction due to inflammatory and biochemical inducers. It comes to an increased production of free radical molecules, advanced glycation end products (AGEs), uric acid (urate) crystals, oxidized lipoproteins, homocysteine, and others (cf. Pahwa et al. (2021) Chronic inflammation. Stat Pearls, as of October 1st, 2021).

Typical chronic inflammatory diseases are for example
- autoimmune disorders such as multiple sclerosis, rheumatoid arthritis, juvenile chronic arthritis, polymyalgia rheumatica, autoimmune hemolytic anemia, Sjögren's syndrome, autoimmune hepatitis, systemic lupus erythematosus, rheumatic fever, alopecia, pemphigus, bullous pemphigoid, dermatomyositis, psoriasis, pyoderma gangrenosum, autoimmune thyroiditis, and immune thrombocytopenia;
- allergies, such as asthma, skin rashes, contact dermatitis, e.g. due to poison oak or poison ivy exposure, urticaria, angioedema, anaphylaxis, and severe hypersensitivity reactions;
- cancers such as leukemias, including acute and chronic lymphoblastic leukemias as well as acute and chronic myelogenous leukemias, lymphomas, Hodgkin lymphoma, non-Hodgkin lymphoma, multiple myeloma, tumors of the brain and intracranial tumors;
- adverse reactions associated to cancer therapies, such as chemotherapy and radiation therapy, including but not limited to fatigue, nausea and vomiting;
- inflammatory diseases of the skin, such as skin rashes, eczema, dermatitis, contact dermatitis, atopic dermatitis, urticaria, systemic lupus erythematosus, alopecia, psoriasis, phimosis, granuloma annulare, pemphigus, bullous pemphigoid, pyoderma gangrenosum, shingles, dermatomyositis, keloids and lichen, such as lichen simplex chronicus, lichen planus, lichen sclerosus and lichen sclerosus et atrophicus;
- inflammatory diseases of the respiratory system, such as asthma, bronchitis, laryngitis, croup, severe tuberculosis, chronic obstructive pulmonary disease (COPD), prevention of exacerbations in COPD, cystic fibrosis, lipid pneumonitis, acute respiratory distress syndrome (ARDS) and prophylaxis of infant respiratory distress syndrome (IRDS) as well as of acute shock lung after a trauma;
- inflammatory diseases of the gastrointestinal tract, such as inflammatory bowel disease, ulcerative colitis, cystic fibrosis, and Crohn's disease;
- inflammatory diseases of the urogenital system, such as acute interstitial nephritis, nephrotic syndrome, cystic fibrosis and phimosis;
- inflammatory diseases of the musculoskeletal system, such as bursitis, osteoarthritis, tendonitis, gouty arthritis, epicondylitis, ataxia telangiectasia, plantar fasciitis, mixed connective tissue disease, dermatomyositis, juvenile chronic arthritis, and polymyalgia rheumatica,
- muscular dystrophies, such as such as Duchenne muscular dystrophy, Becker muscular dystrophy, limb girdle dystrophies, dysferlinopathies, Myoshi myopathy 1-3, hereditary inclusion body myopathy (HIBM), distal myopathy with rimmed vacuoles (DMRV), Nonaka distal myopathy, quadriceps-sparing myopathy, spinal and bulbar muscular atrophy (SMA), SMARD1, Werdnig-Hoffmann disease, Kugelberg-Welander disease, Charcot-Marie-Tooth disease, Curschmann-Steinert's disease, proximal myotonic myopathy, PROMM (type 2), Walker-Warburg syndrome, lamin A/C-related congenital muscular dystrophy, Fukuyama congenital muscular dystrophy, congenital muscular dystrophy with partial merosin deficiency, rigid spine muscular dystrophy, congenital muscular dystrophy with primary laminin 2 deficiency, LARGE-related congenital muscular dystrophy, muscle-eye-brain disease, Ullrich congenital muscular dystrophy, Emery-Dreyfuss muscular dystrophy (EMD), facioscapulohumeral muscular dystrophy, oculopharyngeal muscular dystrophy and myofibrillar myopathies types 1 - 6;
- inflammatory diseases of nervous system and sensory organs, such as ocular inflammation, sympathetic ophthalmia, uveitis, meningitis, cluster headaches, ataxia telangiectasia, Bell's palsy, shingles, chronic inflammatory demyelinating polyneuropathy (CIPD), myasthenia gravis, Menière's disease, acute idiopathic tinnitus,
- inflammatory diseases of the cardiovascular system, such as heart failure, pericarditis, postural hypotension, rheumatic fever with carditis, vasculitis, polyarteritis nodosa, panarteritis nodosa, giant-cell arteritis, temporal arteritis, and granulomatosis with polyangiitis;
- further systemic inflammatory diseases, such as sepsis, including but not limited to septic shock, bacteremia, viremia, fungemia, sepsis associated to parasites, Herxheimer reaction, and SIRS (systemic inflammatory response syndrome) as well as further inflammatory response syndromes, such as the acute respiratory distress syndrome (ARDS), the Kawasaki syndrome and the pediatric inflammatory multisystem syndrome temporally associated with SARS-CoV-2 (PIMS-TS); severe infectious diseases, such as tuberculosis, typhus and brucellosis;
- and a great number of further conditions, such as cerebral edema, shock; posttraumatic shock syndrome, posttraumatic stress disorder, sarcoidosis, hypercalcemia, adrenal insufficiency, including primary, secondary and tertiary adrenal insufficiency, adrenogenital syndrome, thyroiditis, and acute mountain sickness;
and to prevent transplant rejection.

The present application refers thus also to a pharmaceutical combination of budesonide and 5-amino-2,3-dihydro-1,4-phthalazinedione or one of its pharmaceutically acceptable salts for use in the treatment of chronic inflammatory diseases. 5-amino-2,3-dihydro-1,4-phthalazinedione sodium salt is preferred.

Chronic respiratory diseases of the lower airways are of special interest for a treatment with a combination of budesonide and 5-amino-2,3-dihydro-1,4-phthalazinedione or one of its pharmaceutically acceptable salts.

These diseases comprise, without being limiting, bronchitis, not specified as acute or chronic; simple and mucopurulent chronic bronchitis; chronic bronchitis; chronic tracheitis; chronic tracheobronchitis; emphysema; chronic obstructive pulmonary disease (COPD); asthma; status asthmaticus; bronchiectasis; sarcoidosis of the lung; pulmonary fibrosis; croup; pulmonary alveolar microlithiasis.

Also of interest is such a combinatory treatment of non-infectious rhinitis and nasal polyps.

COPD is a progressive development of airflow limitation that is not fully reversible. Most COPD patients suffer from three pathological conditions: Bronchitis, emphysema and mucus plugging. This disease is characterized by a slowly progressive and irreversible decrease in forced expiratory volume in the first second of expiration (FEV1), with relative preservation of forced vital capacity (FVC). In both asthma and COPD there is a significant but distinct remodeling of the airways. Most of the airflow obstruction is due to two major components, alveolar destruction (emphysema) and small airways obstruction (chronic obstructive bronchitis). COPD is mainly characterized by profound mucus cell hyperplasia. A primary characteristic of COPD is neutrophil infiltration into the patient's lungs. Elevated levels of proinflammatory cytokines, like TNF-alpha, and especially chemokines like interleukin-8 (IL-8) play a prominent role in the pathogenesis of COPD. Platelet thromboxane synthesis was found to be enhanced in COPD patients. Most of the tissue damage is caused by activation of neutrophils followed by their release of matrix metalloproteinases, and increased production of ROS and RNS.

Emphysema describes the destruction of the lung architecture with enlargement of the airspaces and loss of alveolar surface area. Lung damage is caused by weakening and breaking the air sacs within the lungs. Several adjacent alveoli may rupture, forming one large space instead of many small ones. Larger spaces can combine into an even bigger cavity, called a bulla. As a result, natural elasticity of the lung tissue gets lost, leading to overstretching and rupture, thus minimizing lung compliance. There is also less pull on the small bronchial tubes, which can cause their collapse and obstruct the airflow. Air not exhaled before the next breath cycle gets trapped in the lungs, leading to shortage of breath.

The sheer effort it takes to force air out of the lungs upon exhaling is exhausting for the patients.

The most common symptoms of COPD include shortness of breath, chronic cough, chest tightness, greater effort to breathe, increased mucus production and frequent clearing of the throat. Patients become unable to perform their usual daily activities.

Long-term smoking is the most common cause of COPD, responsible for 80-90 % of all cases. Other risk factors are heredity, second-hand smoke, air pollution, and a history of frequent childhood respiratory infections. COPD is progressive and sometimes irreversible; there is currently no cure.

The clinical development of COPD is typically described in three stages:
Stage 1: Lung function (as measured by FEV1) is greater than or equal to 50% of predicted normal lung function. There is minimal impact on health-related quality of life. Symptoms may progress during this stage, and patients may begin to experience severe breathlessness, requiring evaluation by a pulmonologist.
Stage 2: FEV1 lung function is 35 to 49% of predicted normal lung function, and there is a significant impact on health-related quality of life.
Stage 3: FEV1 lung function is less than 35% of predicted normal lung function, and there is a profound impact on health-related quality of life.

Symptomatic pharmaceutical therapy includes the administration of bronchodilators, glucocorticoids and PDE4 inhibitors. Suitable bronchodilators are e.g. beta-2 adrenergic agonists such as the short-acting fenoterol and salbutamol as well as the long-acting salmeterol and formoterol, muscarinic anticholinergics such as ipratropium bromide and tiotropium bromide, and methylxanthines such as theophylline.

Suitable glucocorticoids include inhalatory glucocorticoids such as budesonide, beclomethasone and fluticasone, orally administered glucocorticoids such as prednisolone and intravenously administered glucocorticoids such as prednisolone.

A suitable PDE (phosphodiesterase) 4 inhibitor is roflumilast.

In the scope of the present application also asthma is of particular interest. Asthma is a chronic inflammatory disease of the lower airways. It is mainly characterized by recurring symptoms such as reversible airflow obstruction and easily triggered bronchospasms. Symptoms include episodes of wheezing, coughing, chest tightness and dyspnea.

Asthma is thought to be caused by a combination of genetic and environmental factors. Environmental factors include exposure to air pollution and allergens. Other potential triggers may be iatrogenic.

Asthma is clinically classified into intermittent, mild persistent, moderate persistent and severe persistent, based on the frequency of symptoms. The most important parameters are FEV1 and peak expiratory flow rate.

Until now asthma cannot be cured. For long-term therapy, symptoms such as status asthmaticus can be prevented by avoiding triggers, such as allergens and irritants, and pharmacologically by the use of inhaled corticosteroids. Long-acting beta-2 agonists (LABA) or antileukotriene agents may be used additionally. The most common inhaled corticosteroids include beclomethasone, budesonide, fluticasone, mometasone and ciclesonide. Suitable LABA include salmeterol and formoterol. Leukotriene receptor antagonists such as montelukast, pranlukast and zafirlukast are orally administered. Suitable 5-lipooxygenase (5-LOX) inhibitors include meclofenamate sodium and zileuton. In severe stages of asthma intravenous corticosteroids such as prednisolone are recommended.

Acute asthma seizures (status asthmaticus) are best treated with inhaled short-acting beta-2 agonists such as salbutamol. Ipratropium bromide can be inhaled additionally. Intravenously, corticosteroids can be administered.

Inhalatory administration is commonly effected through metered-dose inhalers, respectively dry-powder inhalers.

In the scope of the present application also sarcoidosis of the lung is of particular interest. Sarcoidosis of the lung (interchangeably used herein: pulmonary sarcoidosis, PS) is characterized by abnormal collections of inflammatory cells that form lumps known as granulomas in the lung. The cause of sarcoidosis is unknown. The disease usually begins in the lungs, skin or lymph nodes, and can become manifest throughout the body. The most common symptom is a long-lasting fatigue, even if the disease activity has ceased. Overall malaise, shortness of breath, joint complaints, temperature increase, weight loss and skin complaints may be present. In general, the prognosis is good. Especially the acute form usually causes few problems, as the complaints will gradually decrease by themselves. If sarcoidosis is present in the heart, kidneys, liver and/or central nervous system, or extensively manifest in the lungs the outcome is less favorable. In general, sarcoidosis is classified in four stages determined by chest radiography. However, these stages do not correlate with the grade of severity. 1. bihilar lymphadenopathy (granulomas in the lymph nodes); 2. bihilar lymphadenopathy and reticulonodular infiltrates (granulomas in the lungs); 3. bilateral pulmonary infiltrates (granulomas in the lungs, but not in the lymph nodes); 4. fibrocystic sarcoidosis typically with upward hilar retraction, cystic and bullous changes (irreversible scarring in the lungs i.e., pulmonary fibrosis). Stage 2 and 3 patients often display a chronic progressive disease course.

Symptomatic pharmaceutical therapy of sarcoidosis of the lung includes the administration of corticosteroids such as prednisone and prednisolone, immunosuppressive agents such as TNF-alpha inhibitors (etanercept, adalimumab, golimumab, infliximab), cyclophosphamide, cladribine, cyclosporine, chlorambucil and chloroquine, IL-23 inhibitors such as tildrakizumab and guselkumab, antimetabolites such as mycophenolic acid, leflunomide, azathioprine and methotrexate. In subtypes such as Löfgren's syndrome COX inhibitors such as acetylsalicylic acid, diclofenac or ibuprofen are used. All these active agents are administered systemically until now.

In the scope of the present application also bronchiectasis is of particular interest. Bronchiectasis is believed to be an idiopathic disease. Morphologically, it is characterized by a permanent enlargement of parts of the lower airways. As pathologic conditions, a.o. post-infection, immune deficiency, exaggerated immune response, congenital abnormalities, inflammatory pneumonitis, fibrosis and mechanical obstruction are discussed. Symptoms include chronic cough along with daily production of mucus. Thus, it resembles cystic fibrosis, but without the characteristic gene mutation. Pulmonary function testing results generally show airflow obstruction ranging from moderate to severe. Additional symptoms include dyspnea, coughing up blood, chest pain, hemoptysis, fatigue, and weight loss.

Treatment of bronchiectasis aims at controlling infections and bronchial secretions, relieving airway obstructions, removal of affected portions of lung by surgery or artery embolization. If indicated, antibiotics, in particular macrolide antibiotics are administered. Mucus overproduction can be addressed by mucolytics. Bronchodilators are used for facilitating breathing. Continuous inhaled corticosteroids such as budesonide help to some extent to reduce sputum production, to decrease airway constriction and to prevent disease progression.

In the scope of the present application also pulmonary fibrosis is of particular interest. Herein scars are formed in the pulmonary tissues, leading to serious breathing problems. Scar formation, respectively the accumulation of excess fibrous connective tissue leads to thickening of the walls, thus causing reduced oxygen supply in the blood. The consequence is a chronic progressive dyspnea. Pulmonary fibrosis is often secondary to other pulmonary diseases e.g., in interstitial lung disorders, autoimmune diseases of the lung, inhalation of environmental and occupational pollutants, or certain infections. Otherwise, it is classified as idiopathic pulmonary fibrosis.

Pulmonary fibrosis involves gradual exchange of lung parenchyma with fibrotic tissue. The scar tissue causes an irreversible decrease in oxygen diffusion capacity, resulting in stiffness or decreased compliance of the lung. Pulmonary fibrosis is perpetuated by aberrant wound healing.

Up to now, there is no general pharmaceutical treatment of lung fibrosis. Some subtypes are responsive to corticosteroids such as prednisone, to anti-fibrotic agents such as pirfenidone and nintedanib, or to immunosuppressive agents, such as cyclophosphamide, azathioprine, methotrexate, penicillamine, and cyclosporine.

The present application refers thus also to a pharmaceutical combination of budesonide and 5-amino-2,3-dihydro-1,4-phthalazinedione or one of its pharmaceutically acceptable salts for use in the treatment of a chronic respiratory disease selected from chronic obstructive pulmonary disease, asthma, croup, non-infectious rhinitis and nasal polyps. 5-amino-2,3-dihydro-1,4-phthalazinedione sodium salt is preferred.

In the scope of the present disclosure also inflammatory bowel diseases (IBD) are of particular interest. IBD are recurrent (relapsing) or continuously progressive inflammatory disorders of the intestinal tract. The most common IBD are Crohn's disease and colitis ulcerosa. Less frequent are collagenic and lymphocytic colitis, two forms of microscopic colitis.

Crohn's disease is generally regarded as an autoimmune disease. Typical symptoms are bellyache and diarrhea (sometimes bloody feces). Pain is mostly located in the right abdomen and occur often post-prandially. Further symptoms include fever, weight loss, lack of appetite, nausea and vomiting. Anal fissures, fistulae and abscesses may occur. Laboratory analysis usually reveals an increase in leucocytes and anemia. The disease course is characterized by recurring flare-ups that endure several weeks. Ca. 20% of the cases become manifest in the colon, ca. 30% in the ileum and ca. 50% are ileocolic. Typically, granulomas can be found at the inflamed sites. A lead pipe pattern indicative for a stenosis can occur in the colon. Ca. 50% of the patients display extraintestinal symptoms such as arthralgia, arthritic disorders, erythema nodosum and pyoderma gangraenosum. The etiology of Crohn's disease is not finally resolved. Some patients may have a genetic predisposition. The most prominent gene defect associated with Crohn's Disease is a frameshift in the NOD2 gene. A malfunction of the innate immune system is presumed. The disease becomes manifest when the adaptive immune system tries to compensate for the deficient innate immune system. An impaired cytokine secretion by macrophages has been described, which contributes to impaired innate immunity and leads to a sustained microbial-induced inflammatory response in the colon, where the bacterial load is high. Alternatively, it is presumed that the inflammation in Crohn's disease is caused by an overshoot of the Th1 and Th17 cytokine response.

Apart of symptomatic treatment, flare-ups are treated pharmaceutically with glucocorticoids such as budesonide and prednisone, sulfasalazine and the anti-TNF-a antibodies infliximab, adalimumab and certolizumab are administered. Metronidazole and ciprofloxacin are in use for the treatment of fistulae. In severe cases a resection of the affected intestinal segment may be necessary. During remission periods immunosuppressive drugs such as azathioprine, 6-mercaptopurine, mesalamine (mesalazine, 5-aminosalicylic acid (5-ASA)) and methotrexate (MTX) are used as well as the anti-TNF-a antibodies infliximab, adalimumab and certolizumab, the anti-IL-12/IL-23 antibody ustekinumab and the integrin antagonists vedolizumab and natalizumab.

Colitis ulcerosa is a chronic inflammatory bowel disease that becomes manifest in the colon only. Prevalence in industrialized countries is 160 - 250 per 100,000 inhabitants. Colitis ulcerosa is an autoimmune disease characterized by T-cell infiltration into the colon (Ko et al. (2010) Mol Ther 18: 1365-1372). Genetical factors are probable. It is presumed that the transcription factor NF-κB that controls the expression of pro-inflammatory proteins is permanently activated. The inflammation is limited to the intestinal mucosa and the submucosa. This disorder is characterized by relapsing diarrhea, gastrointestinal bleedings, abdominal pain and colics. Patients suffer from fecal incontinence, increased frequency of bowel movements, constrained defecation and physical weakness. Frequent extraintestinal symptoms are amongst others erythema nodosum, osteopenia and sacroiliitis. In mild forms of colitis ulcerosa edematous swellings of the intestinal mucosa occur. Medium severe forms are characterized by mild bleedings and ulceration. In severe disease courses an extensive ulceration leads to a loss of mucosa. Typically, cryptic abscesses occur. Pseudopolyps are frequent. The disease course of colitis ulcerosa consists of alternating flare-ups (relapses) and remission periods. After a long disease course there is an increased risk for malign neoplasms in the colon.

Pharmaceutical treatment during flare-ups encompasses glucocorticoids such as budesonide that can be administered rectally as an enema or a foam or systemically (orally and intravenously). During remission periods mesalamine (5-ASA) is mostly prescribed. The anti-TNF-α antibodies infliximab, adalimumab and golimumab and immunosuppressive agents such as azathioprine are used in more severe forms. Further medications for severe forms include calcineurin inhibitors such ciclosporin A and tacrolimus as well as JAK kinase inhibitors such as tofacitinib. In very severe cases also a colectomy may become necessary.

The present application refers thus also to a pharmaceutical combination of budesonide and 5-amino-2,3-dihydro-1,4-phthalazinedione or one of its pharmaceutically acceptable salts for use in the treatment of an inflammatory bowel disease selected from Crohn's disease, colitis ulcerosa and microscopic colitis. 5-amino-2,3-dihydro-1,4-phthalazinedione sodium salt is preferred.

Autoimmune hepatitis is a rare acute or chronic inflammatory autoimmune disease of the liver. T cells attack antigens on the hepatic cells and induce thus a hepatitis. Environmental toxins, bacterial antigens (e.g., from *Salmonella sp*.) and viral infections (hepatitis A, B, C and D as well as measles and herpes viruses) are discussed as triggers of autoimmune hepatitis. Typical symptoms include fatigue, nausea, loss of appetite, fever, joint pain and icterus (jaundice). Extrahepatic symptoms are centered on autoimmune inflammations of the thyroid, the vessels, the colon, the pleura and the skin. Further, autoimmune-induced anemia is frequent. In mild forms, leucocyte infiltration is limited to the portal tract, in medium-to-severe forms the hepatic lobules are affected too. There is a necrotization of the affected tissue, at later (or more severe) stages also a fibrosis and finally a liver cirrhosis. The prevalence is 10 - 20 / 100,000 in the Western world. Interestingly, women are four times more often affected than men. The disease usually becomes manifest before 30 years of age.

If not treated adequately, autoimmune hepatitis takes a lethal course. Pharmaceutic therapy focuses on glucocorticoids such as prednisone and budesonide. The glucocorticoids are often combined with the immunosuppressive agent azathioprine for reducing the feared side effects of the glucocorticoids. In autoimmune hepatitis these side effects are mainly osteoporosis and Cushing syndrome. Second-line medications include cyclophosphamide, tacrolimus, methotrexate (MTX) and mycophenolate mofetil. In most cases pharmaceutical therapy is life-long. If all medications fail the last option is a liver transplantation.

The present application refers thus also to a pharmaceutical combination of budesonide and 5-amino-2,3-dihydro-1,4-phthalazinedione or one of its pharmaceutically acceptable salts for use in the treatment of autoimmune hepatitis. 5-amino-2,3-dihydro-1,4-phthalazinedione sodium salt is preferred.

Unless otherwise stated, any technical or scientific term used in the present invention has the meaning that a man skilled in the relevant technical art will attribute to them.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" in regard with the substance of the invention or any other active substance mentioned in the description refers to causally occurring beneficial consequences in the organism to which said substance has been administered before.

According to the disclosure the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of a congenital muscular dystrophy by stopping or decelerating the increase of disabilities during the course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to a congenital muscular dystrophy. It refers particularly to medical conditions of a patient in which the manifestation of such symptoms is expected to occur in the far or near future with a reasonable probability.

The terms "subject" and "patient" comprise individuals suffering from disease symptoms or disabilities related to a congenital muscular dystrophy wherein said diagnosis is either approved or suspected. Individuals are mammals, in particular humans.

In another aspect of the invention a pharmaceutical composition containing budesonide and 5-amino-2,3-dihydro-1,4-phthalazinedione or one of its pharmaceutically acceptable salts and at least one pharmaceutically acceptable excipient is disclosed.

In particular, the present application relates to a pharmaceutical composition containing budesonide and 5-amino-2,3-dihydro-1,4-phthalazinedione or one of its pharmaceutically acceptable salts and at least one pharmaceutically acceptable excipient for use in the treatment of chronic inflammatory diseases.

Application forms of the compositions according to the invention as outlined above comprise but are not limited to oral, parenteral, intravenous, intraarterial, by inhalation, by intubation, intramuscular, topical, transdermal, subcutaneous, intradermal, transmucosal, sublingual, buccal, conjunctival, intravaginal, rectal or nasal administration.

Preferred is a pharmaceutical composition according to the invention, wherein said pharmaceutical composition is suitable for intravenous, oral, sublingual, inhalatory, rectal, topical or dermal administration.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the disclosure include, carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents (antiadherents), permeation enhancers, sorbents, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the disclosure may use carriers known in the art such as diluents (e.g., water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulfite caramel, ammonia caramel, sulfite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylamino] propane), ethylene diamine, ACES (2-[(amino-2-oxoethyl)amino]ethanesulfonic acid), imidazole, MOPS (3-(N-morphino) propanesulfonic acid), diethyl malonic acid, TES (2-[tris(hydroxymethyl)methyl]aminoethanesulfonic acid), HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and *N,N*-bis-(2-hydroxyethyl)-2-aminoethanesulfonic acid (BES). Another group of preferred buffers are glycine, glycyl-glycine, glycyl-glycyl-glycine, *N,N*-bis-(2-hydroxyethyl)glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy lysine, o-phosphoserine, gamma-carboxyglutamate, [epsilon]-*N*-acetyl lysine, [omega]-*N*-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisole, butylated hydroxytoluene, fumaric acid or propyl gallate. Preferred is the use of sodium metabisulfite, alpha-tocopherol and ascorbyl palmitate. A potent antioxidant is inhaled carbon monoxide (CO).

Tablets or pills are usually coated, i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the active substance. The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g. sodium hydroxide, hydrochloric acid, buffer substances such as sodium dihydrogen phosphate or disodium hydrogen phosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs for handling minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthan gum, gum arabic or any combination thereof.

Anti-caking agents (antiadherents) can be added to a supplement or a composition of supplements for preventing the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamot, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonen, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonen, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the disclosure all the aforementioned excipients and classes of excipients can be used without limitation alone or in any conceivable combination thereof, as long as the inventive use is not thwarted, toxic actions may occur, or respective national legislations are infracted.

According to the invention it is possible to administer the pharmaceutical combination of the invention, wherein 5-amino-2,3-dihydro-1,4-phthalazinedione or one of its pharmaceutically acceptable salts is either administered as add-on to budesonide or within the same pharmaceutical composition with budesonide.

In another aspect of the invention the present application relates to a combination of budesonide and 5-amino-2,3-dihydro-1,4-phthalazinedione or one of its pharmaceutically acceptable salts, respectively a pharmaceutical composition according to the disclosure for use in a formulation for oral administration.

Pharmaceutical formulations suitable for oral dosage forms of a combination of budesonide and 5-amino-2,3-dihydro-1,4-phthalazinedione or one of its pharmaceutically acceptable salts, respectively a pharmaceutical composition according to the disclosure may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner, e.g., an edible carbohydrate such as starch or mannitol. A flavor, preservative, dispersant or colorant can also be present.

Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the disclosure can also be combined with a free-flowing inert excipient and then pressed directly to give tablets without carrying out the granulation or dry-pressing steps.

In another aspect of the invention a combination of budesonide and 5-amino-2,3-dihydro-1,4-phthalazinedione or one of its pharmaceutically acceptable salts, respectively a pharmaceutical composition according to the disclosure is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise for improving the availability of the medication after intake of the capsule. Additionally, suitable binding agents and/or colorants can be added to the mixture, if desirable or necessary.

In another aspect of the invention a combination of budesonide and 5-amino-2,3-dihydro-1,4-phthalazinedione or one of its pharmaceutically acceptable salts, respectively a pharmaceutical composition according to the disclosure is included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

In another aspect of the invention a combination of budesonide and 5-amino-2,3-dihydro-1,4-phthalazinedione or one of its pharmaceutically acceptable salts, respectively a pharmaceutical composition according to the disclosure is included in chewable tablets or hard caramels. Herein the substance is integrated into the matrix of the tablets or caramels.

In another aspect of the invention the present application the pharmaceutical combination according to the invention is formulated in a retard formulation, i.e. a formulation with a delayed release of at least one of budesonide or 5-amino-2,3-dihydro-1,4-phthalazinedione or one of its pharmaceutically acceptable salts. They are also known as sustained release (*SR*), extended release (*ER, XR*) or controlled/continuous release (*CR*) forms. Suitable formulations and carriers are known to a person skilled in the art (Kleinsorge (1995) Retardformulierungen in der medikamentösen Therapie. Leipzig, Barth 8th ed.). Most commonly, the at least one active agent is embedded in a matrix of insoluble substances like acrylics or chitin. The active agent must thus find its way out through orifices in the matrix. In some formulations, there is a laser-drilled hole on one side and opposite to it a porous membrane. The gastric fluid attacks this porous membrane, flows in and pushes the active agent through the drilled hole on the opposite side. In other formulations, the active agent dissolves inside the matrix swelling thereupon and forming a gel. Then the active agent is released through the pores of the gel. Other examples include specifically coated tablets resistant to gastric fluid, retard capsules containing retard pellets of the active agent that are going to be released after the dissolution of the capsule casing, multiple unit pellet systems (MUPS), oral osmotic systems, resonates, coacervation and micro-encapsulation. With the use of such a retard formulation the release site of a drug and its pharmacokinetics can be controlled. For example, it is often desirable that a dosage form of an active agent is not dissolved before reaching a certain point of the intestines. As the pH changes along the way through the intestines, the dissolution process may be engineered to be pH dependent. In therapeutic applications in which the absorption of an active agent through the intestinal mucosa shall be facilitated in order to augment its bioavailability it may be preferable not to use a salt of an active agent but its neutral form.

In another aspect of the invention the present application relates to a combination of budesonide and 5-amino-2,3-dihydro-1,4-phthalazinedione or one of its pharmaceutically acceptable salts, respectively a pharmaceutical composition according to the disclosure for use in the treatment of a chronic respiratory disease of the lower airways in a formulation for inhalatory administration.

For an effective inhalatory treatment it is advantageous that budesonide as well as 5-amino-2,3-dihydro-1,4-phthalazinedione or one of its pharmaceutically acceptable salts, a pharmaceutical composition according to the disclosure or a pharmaceutical combination according to the disclosure reaches the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, PARI eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bialystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the invention the present application relates to a combination of budesonide and 5-amino-2,3-dihydro-1,4-phthalazinedione or one of its pharmaceutically acceptable salts, respectively a pharmaceutical composition according to the disclosure for use in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD do not deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another aspect of the invention the present application refers also to a method for producing an aerosol according to the disclosure, comprising the following steps:
a) filling 0.1 ml to 5 ml of an aqueous solution containing a combination of budesonide and 5-amino-2,3-dihydro-1,4-phthalazinedione or one of its pharmaceutically acceptable salts and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, *The Aerosol Society*: **DDL2019;** Gardenshire et al. (2017) A Guide to Aerosol Delivery Devices for Respiratory Therapists, 4th ed.).

Thus, the aforementioned method is also disclosed with said vibration frequency ranges.

The method according to the disclosure is thus characterized in that at least 80 % in weight, preferred at least 85 % in weight, most preferred at least 90 % in weight of the combination of budesonide and 5-amino-2,3-dihydro-1,4-phthalazinedione or one of its pharmaceutically acceptable salts contained in said aqueous solution are nebulized in the generated aerosol.

The method of the invention is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes' time span 95 % of the substance provided in the aqueous solution could be nebulized. This is an ideal time span for a high patient compliance.

Therefore, the method according to the disclosure is thus characterized in that at least 80 % of the generated aerosol are produced during three minutes after starting nebulization in the mesh nebulizer, preferred at least 85 % and most preferred at least 90 %.

While pharmaceutically active agents are usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit comprising a mesh nebulizer and a pharmaceutically acceptable container with an aqueous solution containing an effective amount of a combination of budesonide and 5-amino-2,3-dihydro-1,4-phthalazinedione or one of its pharmaceutically acceptable salts and optionally at least one pharmaceutically acceptable excipient.

In an alternative kit the combination of budesonide and 5-amino-2,3-dihydro-1,4-phthalazinedione or one of its pharmaceutically acceptable salts, respectively a pharmaceutical composition according to the disclosure is not provided in form of an aqueous solution but in two separated containers, one for a solid form for the active agents and the other for an aqueous solution. The final aqueous solution is freshly prepared by solving the active agent in the final solution. Thereupon the final aqueous solution is filled into the nebulization chamber of the mesh nebulizer. These two containers can be completely separated containers e.g., two vials, or e.g., a dual-chamber vial. For solving the active agent e.g., a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with effective dosages of a solid form of budesonide and 5-amino-2,3-dihydro-1,4-phthalazinedione or one of its pharmaceutically acceptable salts, respectively a pharmaceutical composition according to the disclosure, wherein optionally at least one pharmaceutically acceptable excipient is contained in the first pharmaceutically acceptable container and/or the second pharmaceutically acceptable container.

The aerosol generated by the method according to the disclosure is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless-steel injection needle would be in the range of 14 to 27.

In yet another aspect of the invention a combination of budesonide and 5-amino-2,3-dihydro-1,4-phthalazinedione or one of its pharmaceutically acceptable salts, respectively a pharmaceutical composition according to the invention is disclosed, wherein said substance, composition or combination is applied in form of liposomes, micelles, multilamellar vesicles or a cyclodextrin complex.

In yet another aspect of the invention the present application relates to a combination of budesonide and 5-amino-2,3-dihydro-1,4-phthalazinedione or one of its pharmaceutically acceptable salts, respectively a pharmaceutical composition according to the disclosure in a liquid dosage form.

The present application discloses also the parenteral administration of a combination of budesonide and 5-amino-2,3-dihydro-1,4-phthalazinedione or one of its pharmaceutically acceptable salts, respectively a pharmaceutical composition according to the disclosure in the form of a intravenous injection solution, intraarterial injection solution or intraperitoneal injection solution.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

In yet another aspect of the invention a combination of budesonide and 5-amino-2,3-dihydro-1,4-phthalazinedione or one of its pharmaceutically acceptable salts, respectively a pharmaceutical composition according to the invention is disclosed, wherein said combination or said composition is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In general, an aqueous solution or a physiological saline solution is preferred. In case of a poorly soluble pharmaceutical agent according to the disclosure also ethanol or ethanol/water mixtures can be used.

Further suitable liquid dosage forms are drops, gels and hydrogels.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as a colloidal gel in which water is the dispersion medium. A three-dimensional solid results from the hydrophilic polymer chains being held together by cross-links. Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

In yet another aspect of the invention the present application relates to a combination of budesonide and 5-amino-2,3-dihydro-1,4-phthalazinedione or one of its pharmaceutically acceptable salts, respectively a pharmaceutical composition according to the disclosure in a formulation for sublingual tablets.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

For avoiding a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

For topical applications containing a combination of budesonide and 5-amino-2,3-dihydro-1,4-phthalazinedione or one of its pharmaceutically acceptable salts, respectively a pharmaceutical composition according to the disclosure for use according to the invention creams, emulsions, lotions, gels, hydrogels, pastes, powders, ointments, liniment, films, liposomes, dermal patches, transdermal patches, transdermal sprays or suspensions are suitable.

Permeation enhancers are often used in topical dosage forms. Suitable permeation enhancers comprise all pharmaceutically acceptable permeation enhancers known in the art, such as, without being limiting, azones such as laurocapran, 1-dodecylazacycloheptan-2-one; sulfoxides such as dimethyl sulfoxide, DMAC, DMF; pyrrolidones such as 2-pyrrolidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transkarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64); citric acid esters such as triethyl citrate; hydrophobin polypeptides; alpha-bisabolol; dimethyl isosorbide (Arlasolve^{®} DMI); ethoxydiglycol. Preferred is 1,2-propandiol.

Typical examples for preservatives suitable for topical applications are e.g. benzyl benzoate, benzoic acid, benzyl alcohol, benzalkonium chloride, *N*-cetyl-*N-N*-trimethylammonium bromide (Cetrimid, Merck), chlorhexidine, chlorobutanol, chlorocresol, imidurea, parabens such as methyl, ethyl, propyl or butyl paraben, sodium methylparaben, sodium propylparaben, potassium sorbate, sodium benzoate, sodium propionate, phenol, phenoxyethanol, phenylethyl alcohol, phenylmercuriacetate, phenylmercuriborate, phenylmercurinitrate, sorbic acid or Thiomersal (sodium methylmercurithiosalicylate). Preferred are methylparaben, propylparaben as well as sodium methylparaben and sodium propylparaben.

The addition of antioxidants is particularly preferable in topical dosage forms. Suitable examples for antioxidants include sodium metabisulfite, alpha-tocopherol, ascorbic acid, maleic acid, sodium ascorbate, ascorbyl palmitate, butylated hydroxyanisole, butylated hydroxytoluene, fumaric acid or propyl gallate. Preferred is the use of sodium metabisulfite.

Suitable pH-regulators for topical dosage forms are e.g., sodium hydroxide, hydrochloric acid, buffer substances such as sodium dihydrogen phosphate or disodium hydrogen phosphate.

Cream preparations may also contain other excipients and additives, such as fatliquors, solvents, consistency enhancers or hydrotropes for improving the flow characteristics. Herein single as well as several substances from the same group of additives or excipients may be present in the mixture.

For producing a dosage form of a suppository containing the pharmaceutical combination according to the invention waxes with a low melting point as well as a mixture of fatty acid glycerides such as cocoa butter are first melted, then budesonide and 5-amino-2,3-dihydro-1,4-phthalazinedione sodium salt are homogenously dispersed under stirring or other mixing methods. The molten homogeneous mixture is transferred to suitable molds and then cooled down until solidification.

A specific form of topical application are enemas. An enema (clyster) is an injection of a medical fluid into the lower bowel of the rectum. For the pharmaceutical combination according to the disclosure they can be used in the treatment of inflammatory bowel diseases. An enema containing budesonide can be produced with a dispersible tablet and a solution for rectal suspension (Entocort^{®} enema). The dispersible tablet can contain the following excipients: Lactose anhydrous, riboflavin sodium phosphate, lactose monohydrate, polyvidone, colloidal anhydrous silica and magnesium stearate. The vehicle may contain sodium chloride, methyl p-hydroxybenzoate, propyl p-hydroxybenzoate and purified water. 5-amino-2,3-dihydro-1,4-phthalazinedione or one of its pharmaceutically acceptable salts can be admixed to the dispersible tablet of a budesonide edema or concomitantly administered via another dosage form. 5-amino-2,3-dihydro-1,4-phthalazinedione sodium salt Form I is preferred.

Another specific form of topical application are rectal foams, in particular in the treatment of inflammatory bowel diseases. They are an alternative to suppositories or enemas. Unwanted systemic side effects in glucocorticoid therapy can thus be avoided or at least mitigated. A composition of a rectal foam of budesonide (UCERIS^{®}) contains cetyl alcohol, citric acid monohydrate, edetate disodium, macrogol stearyl ether, emulsifying wax, polyoxyl (10) stearyl ether, propylene glycol and purified water. As a propellant for rectal application, butane, isobutane, propane, or a mixture thereof can be used (cf. Budenofalk 2mg/dose rectal foam - Summary of Product Characteristics (SmPC) - (emc) (medicines.org.uk), as of October 7th, 2021). 5-amino-2,3-dihydro-1,4-phthalazinedione or one of its pharmaceutically acceptable salts can be admixed to a budesonide rectal foam or concomitantly administered via another dosage form.

Surprisingly, it can be shown that the concomitant administration of a pharmaceutical combination according to the invention not only shows a general supraadditive effect, but also shows this effect over the whole great range of fixed ratios tested (see Example 1).

The term "ratio" or "fixed ratio" hereby refers to any kind of ratio between two components independent of the units used. Such a ratio is valid for weight, weight %, concentration specifications and any other feasible unit in the field of pharmaceuticals. Hence, for example a ratio of budesonide to 5-amino-2,3-dihydro-1,4-phthalazinedione or one of its pharmacologically acceptable salts of 1:10 could for example be implemented as 1mg:10mg, 1mg/kg:10mg/kg, 1mM/10mM or 1%:10% or in any other unit, as long as the ratio itself is 1:10.

The present patent application also refers to a budesonide-sparing agent or a respective pharmaceutical combination comprising 5-amino-2,3-dihydro-1,4-phthalazinedione or one of its pharmacologically acceptable salts, hydrates and solvates and budesonide for use in the prophylaxis and/or treatment of conditions or diseases usually treated with budesonide, wherein the efficacy of the prophylaxis and / or treatment is significantly improved compared to the respective treatment with budesonide alone.

Further, the pharmaceutical combination according to the invention can thus be used in the prophylaxis and/or treatment of conditions or diseases usually treated with budesonide, wherein the components can be used in any ratio.

However, to receive the best effects the medical indication to be treated, the potency of budesonide, the application form and feasibility of the ratio should be considered from a practical point of view, the latter in particular for maintaining patient compliance.

The pharmaceutical combination according to the invention can thus be used in the prophylaxis and/or treatment of conditions or diseases usually treated with budesonide, wherein budesonide and 5-amino-2,3-dihydro-1,4-phthalazinedione or one of its pharmaceutically acceptable salts can be preferably used in any ratio from 1:10 to 1:50,000, more preferably from 1:15 to 1:10,000, still more preferably 1:20 to 1:5,000, most preferably from 1:30 to 1:2,500.

In the following some possible combinations and respective ratios are provided, however, the pharmaceutical combinations according to the invention are not limited to these examples. Depending on application form, indication to be treated and patients personal risk situation budesonide for oral application for example is administered in doses of up to 9 mg/d in severe and acute cases whereas a typically maintenance doses in chronic conditions is 6 mg/d. 5-amino-2,3-dihydro-1,4-phthalazinedione sodium salt has been shown to be very safe, however, due to compliance reasons dosages should not exceed 10 g/d in case of e.g., tablets or capsules.

The pharmaceutical combination according to the invention can thus be used in the prophylaxis and/or treatment of conditions or diseases usually treated with budesonide, wherein budesonide is administered orally, characterized by a ratio of budesonide to 5-amino-2,3-dihydro-1,4-phthalazinedione sodium salt from 1:10 to 1:10,000, preferably 1:15 to 1:5,000, more preferably 1:20 to 1:2,000, most preferably 1:30 to 1:1,000 in severe acute cases, and characterized by a ratio of budesonide to 5-amino-2,3-dihydro-1,4-phthalazinedione sodium salt from 1:15 to 1:10,000, preferably 1:20 to 1:5,000, more preferably 1:30 to 1:2,000, most preferably 1:50 to 1:1,000 for maintenance doses in chronic cases.

The pharmaceutical combination according to the invention can thus be used in the prophylaxis and/or treatment of conditions or diseases usually treated with budesonide, wherein the pharmaceutical combination according to the invention is administered via inhalation, characterized by a ratio of budesonide to 5-amino-2,3-dihydro-1,4-phthalazinedione sodium salt from 1:30 to 1:2,000, preferably 1:50 to 1:1,000, more preferably 1:100 to 1:500, most preferably 1:100 to 1:200 in severe acute cases, and characterized by a ratio of budesonide to 5-amino-2,3-dihydro-1,4-phthalazinedione sodium salt from 1:25 to 1:2,000, preferably 1:30 to 1:1,000, more preferably 1:50 to 1:500, most preferably 1:100 to 1:200 for maintenance doses in chronic cases.

The pharmaceutical combination according to the invention can thus be used in the prophylaxis and/or treatment of conditions or diseases usually treated with budesonide, wherein the pharmaceutical combination according to the invention is administered via a rectal foam, characterized by a ratio of budesonide to 5-amino-2,3-dihydro-1,4-phthalazinedione sodium salt from 1:25 to 1:5,000, preferably 1:40 to 1:2,000, more preferably 1:50 to 1:1,000, most preferably 1:100 to 1:500 for maintenance doses in chronic cases.

However, in case of liquid applications higher doses and/or amounts could be administered without negative impact on patient's compliance.

Due to the supraadditivity of the pharmaceutical combinations of the invention the budesonide dose can be reduced depending on individual patient, indication and ratio of components used. In some cases, dose and ratio adaption over time might become necessary to receive best results.

Thus, the budesonide dosage in the pharmaceutical combination of the invention can be reduced to 80%, preferably 50%, most preferably 20% compared to the dosage when budesonide is administered alone.

The present application refers thus to 5-amino-2,3-dihydro-1,4-phthalazinedione or one of its pharmaceutically acceptable salts for use as budesonide-sparing agent in the prophylaxis and/or treatment of conditions usually treated with budesonide, characterized in that the dose of budesonide can be significantly reduced. Such a significant reduction means a reduction of the budesonide dose to 80%, preferably 50%, most preferably 20% of the original dose.

The present patent application also refers to a pharmaceutical combination comprising 5-amino-2,3-dihydro-1,4-phthalazinedione sodium salt and budesonide for use in the prophylaxis and/or treatment of conditions or diseases usually treated with budesonide, wherein the dose of budesonide can be significantly reduced compared to the respective treatment with budesonide alone. Such a significant reduction means a reduction of the budesonide dose to 80%, preferably 50%, most preferably 20% of the original dose.

In a further aspect, the invention relates to a method of treatment, in which an effective dose of a combination of budesonide and 5-amino-2,3-dihydro-1,4-phthalazinedione or one of its pharmaceutically acceptable salts, respectively a pharmaceutical composition according to the disclosure is administered to a patient in need thereof.

The present disclosure refers likewise to 5-amino-2,3-dihydro-1,4-phthalazinedione or one of its pharmaceutically acceptable salts, hydrates or solvates for use in a method for the treatment and/or prevention of a chronic inflammatory disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of budesonide or one of its pharmaceutically acceptable salts.

The present disclosure refers likewise to 5-amino-2,3-dihydro-1,4-phthalazinedione or one of its pharmaceutically acceptable salts, hydrates or solvates for use in a method for the treatment and/or prevention of a chronic inflammatory disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of budesonide or one of its pharmaceutically acceptable salts, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment and/or prevention of a chronic inflammatory disease in a subject, wherein the method comprises administering to the subject an affective amount of budesonide or one of its pharmaceutically acceptable salts and concomitantly or subsequently an effective amount of 5-amino-2,3-dihydro-1,4-phthalazinedione or one of its pharmaceutically acceptable salts, hydrates or solvates.

The present disclosure refers likewise to 5-amino-2,3-dihydro-1,4-phthalazinedione or one of its pharmaceutically acceptable salts, hydrates or solvates for use in a method for the treatment and/or prevention of a chronic inflammatory disease in a subject, wherein the method comprises administering to the subject an affective amount of one of budesonide or one of its pharmaceutically acceptable salts and concomitantly or subsequently an effective amount of 5-amino-2,3.-dihydro-1,4-phthalazinedione or one of its pharmaceutically acceptable salts, hydrates or solvates, wherein 5-amino-2,3-dihydro-1,4-phthalazinedione is a sodium salt.

### EXAMPLES

### Example 1: Effects of a combination of budesonide and 5-amino-2,3-dihydro-1,4-phthalazinedione sodium salt on LPS-stimulated RAW264.7 cells

In order to test whether a combination of budesonide and 5-amino-2,3-dihydro-1,4-phthalazinedione sodium salt shows a synergistic effect and/or allows for a booster effect, the following experiments are carried out with 5-amino-2,3-dihydro-1,4-phthalazinedione sodium salt Form I, as defined earlier in the description. Raw264.7 cells (leukemia transformed monocytic-macrophage cell line derived from mouse ascitic fluid) are grown in Dulbecco's Modified Eagle's Medium enriched with glutamine and fetal calf serum and maintained at 37°C and 5% CO₂.

RAW264.7 cells are pre-incubated with 5-amino-2,3-dihydro-1,4-phthalazinedione sodium salt (dose range 0.025 mM - 1 mM) and the following concentrations of budesonide (1, 0.1, and 0.01 µM, as well as sub-threshold concentrations of serial two-fold dilutions spanning a range from 0.01 - 0.0003 µM) for 1 h before LPS (lipopolysaccharides) stimulation (100 ng/ml). After 24 hours interleukin 6 (IL-6) levels are determined in supernatants by an ELISA assay and compared with supernatants from cells treated with 5-amino-2,3-dihydro-1,4-phthalazinedione sodium salt or budesonide alone, respectively.

Two experimental parts are performed. In part 1 of the experiment budesonide is used at three ten-fold scalar concentrations (1, 0.1 and 0.01 µM) and incubated with two-fold scalar concentrations of 5-amino-2,3-dihydro-1,4-phthalazinedione sodium salt (1 - 0.025 mM range). Part 2 of the experiment is performed to further demonstrate the effect of the combination of budesonide and 5-amino-2,3-dihydro-1,4-phthalazinedione sodium salt on IL-6 levels when suboptimal doses of budesonide are used. Hence, in experimental part 2 six serial two-fold dilutions of budesonide starting from 0.01 µM as the highest concentration are administered together with 5-amino-2,3-dihydro-1,4-phthalazinedione sodium salt.

## Claims

1. Pharmaceutical combination consisting of budesonide and 5-amino-2,3-dihydro-1,4-phthalazinedione or one of its pharmaceutically acceptable salts.

2. Pharmaceutical combination according to claim 1, wherein said pharmaceutically acceptable salt is 5-amino-2,3-dihydro-1,4-phthalazinedione sodium salt.

3. Pharmaceutical combination as defined in any one of claims 1 or 2 for use for glucocorticoid-sparing.

4. Pharmaceutical combination as defined in any one of claims 1 or 2 for use for reducing or avoiding unwanted budesonide side effects.

5. Pharmaceutical combination as defined in any one of claims 1 or 2 for use in the treatment of chronic inflammatory diseases.

6. Pharmaceutical combination for use according to claim 5, wherein said chronic inflammatory disease is a chronic respiratory disease selected from chronic obstructive pulmonary disease, asthma, croup, non-infectious rhinitis and nasal polyps.

7. Pharmaceutical combination for use according to claim 5, wherein said chronic inflammatory disease is an inflammatory bowel disease selected from Crohn's disease, colitis ulcerosa and microscopic colitis.

8. Pharmaceutical combination for use according to claim 5, wherein said chronic inflammatory disease is autoimmune hepatitis.

9. Pharmaceutical combination for use according to any one of claims 5 to 8, wherein 5-amino-2,3-dihydro-1,4-phthalazinedione or one of its pharmaceutically acceptable salts is either administered as add-on to budesonide or within the same pharmaceutical composition with budesonide.

10. Pharmaceutical composition containing budesonide and 5-amino-2,3-dihydro-1,4-phthalazinedione or one of its pharmaceutically acceptable salts and at least one pharmaceutically acceptable excipient.

11. Pharmaceutical composition according to claim 10 for use in the treatment of chronic inflammatory diseases.

12. Pharmaceutical composition according to any one of claims 10 or 11, wherein said pharmaceutical composition is suitable for intravenous, oral, sublingual, inhalatory, rectal, topical or dermal administration.

13. Pharmaceutical composition according to any one of claims 10 to 12 for oral application, wherein a dosage form is selected from tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

14. Pharmaceutical composition according to any one of claims 10 to 13, wherein said at least one pharmaceutically acceptable excipient is selected from a group comprising carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents, permeation enhancers, sorbents, foaming agents, anti-foaming agents, opacifiers, fatliquors, consistency enhancers, hydrotropes, aromatic and flavoring substances.

15. A method of treatment of a chronic inflammatory disease in which an effective dose of a pharmaceutical combination consisting of budesonide and 5-amino-2,3-dihydro-1,4-phthalazinedione or one of its pharmaceutically acceptable salts or a pharmaceutical composition as defined in claim 8 is administered to a patient in need thereof.
